# EUROPEAN PATENT SPECIFICATION

(11) **EP 0 606 934 B1**
(45) Date of publication and mention of the grant of the patent: **20.08.1997**
(21) Application number: 94200013.4
(22) Date of filing: 01.02.1988
(51) Int. Cl.: C07C 47/58, C07C 45/54, C07C 45/28

(54) **Hydrolysis of curcumin**
Hydrolyse von Curcumin
Hydrolyse du curcumin

(30) Priority: 04.02.1987 US 10902; 20.11.1987 US 123137
(43) Date of publication of application: 20.07.1994
(62) Divisional of application: 88902311.5
(73) Proprietor: FRIES & FRIES, INC., Cincinnati, Ohio 45216 (US)
(72) Inventor: Dolfini, Joseph E., Ohio 45241 (US); Glinka, Jerome, Ohio 45224 (US)
(74) Representative: Findlay, Alice Rosemary

(56) References cited:
- DE-C- 875 512

## Description

This application is a divisional of European Patent Application No. 0346383 entitled "Hydrolysis of Activated Olefinic Ketones and Aldehydes".

In the field of organic chemistry, reaction products different in form from their starting materials can be obtained by means of a large number of reaction sequences. Organic starting materials particularly amenable to reaction are alkene compounds, otherwise known as olefin. Olefin are characterized by the presence of one or more carbon-carbon double bonds. Reactions involving olefin usually occur at the carbon-carbon double bonds. The double bond can be eliminated by addition of atoms to the olefin molecule. The double bond can also be shifted from one carbon-carbon couple to another within the molecule. The double bond can also be cleaved to form two or more smaller molecules from one olefin molecule having one or more double bonds. Cleavage is accomplished by a small number of reaction sequences such as ozonolysis, oxidation and the like. Generally, cleavage occurs by the addition of oxygen to the double bond carbons which then destroys the bond between the carbons.

The presence of a substituent group on the olefin compound sometimes permits the double bond to be broken under less vigorous conditions. One such substituent is the carbonyl group, which consists of a carbon doubly bonded to oxygen. A carbonyl group having its double bond conjugated with the olefin double bond can activate the olefin double bond and cause it to break in the presence of alkaline catalyst, heat and water. This reaction is known as a reverse or retro-aldol condensation which are subject to side reactions and reaction product condensations. In the presence of only heat and water, typically no reaction occurs.

One reference has been found which describes the cleavage of a substituted conjugated cyclohexanone molecule in the presence of heat, pressure and water without any added alkaline catalyst. The reaction as cited in Annalen, Vol. 289, p.337 (1896), involved the formation of acetone and 3-methyl-cyclohexanone in an autoclave at 250°C from pulegone and water.

It is believed that pulegone underwent reaction under the above conditions because the position of the substituents on the cyclohexyl ring caused a destabilization of the olefin, permitting reaction to occur.

U.S. Patent No. 4617419 describes the production of benzaldehyde and acetaldehyde from cinnamaldehyde by a "retro-aldol" reaction. The cinnamaldehyde may be naturally existing cinnamaldehyde or it may be recovered from natural sources, e.g. by distillation. The reaction is conducted at a temperature of between 40°C to about 150°C, a pressure from about 0.2 atmospheres to about 10 atmospheres and in the presence of a base. The mole ratio of base to cinnamaldehyde may vary from 0.1:1 to 4:1. The reaction may take place in the presence of C₁-C₅ alcohols and/or water.

DE-A-875 512 describes the hydrolyzation of unsaturated ketones which contain a carbon-carbon double bond in the neighbouring position to the carbonyl group in the presence of water at elevated temperatures and pressures, whereby the double bond conjugated with the carbonyl group in the starting material is cleaved.

In accordance with the invention, there is provided a method of hydrolysing curcumin to produce flavour compounds, in particular vanillin, in which the curcumin is heated in the presence of water at a temperature and pressure sufficient to effect the reaction and thereby produce the compound. The hydrolysis reaction is preferably conducted at substantially neutral pH and, very preferably, the method comprises conducting the reaction at substantially neutral pH in the presence of water, and heating at a superatmospheric pressure sufficient to effect the hydrolysis reaction.

As is disclosed in the above-mentioned parent European Patent Application No. 0346383, it has been found that acyclic unsaturated ketones and aldehydes having a carbon-carbon double bond conjugated with the carbonyl double bond undergo hydrolysis in the presence of heat, pressure and water which breaks the conjugated carbon-carbon double bond and forms new carbonyl-containing reaction products. Also, the hydrolysis reaction causes cleavage of a substantial portion of the starting material in practical yield. The hydrolysis of the alpha, beta-unsaturated compound actually proceeds contrary to the normal acid or base catalyzed aldol reaction sequence. The favoured sequence would produce an alpha, beta-unsaturated compound from the carbonyl-containing compounds with formation of water. One of the advantages of this invention is the production of natural flavours from natural sources simply under the reaction conditions of temperature, pressure and water with no catalysts.

The method of hydrolysing activated olefinic aldehydes to produce carbonyl-containing compounds at substantially neutral pH is the subject of the parent European Patent Application No. 0346383.

U.S. Patent No. 4709098 describes that the reaction proceeds in the presence of water with a temperature in the range of about 200°C to about 300°C and pressure in the range of about 225 to about 1250 psi (1.5 x 10⁶ to about 8.6 x 10⁶ N/m²). As is disclosed in parent European Patent Application No. 0346383, it has also been found that the hydrolysis reaction can be effectively conducted at even higher temperatures and pressures at about 325°C and 2000 psi (13.7 x 10⁶ N/m²). It is preferred to conduct the reaction at a temperature of at least about 200°C and a pressure of at least about 225 psi (1.5 x 10⁶ N/m²). Minor variations in temperature or pressure during the reaction have no significant effect on the generation of product and therefore can be tolerated. Thus, broadly, the method of parent European Patent Application No. 0346383 comprises hydrolysis of the above-mentioned class of compounds at substantially neutral pH in the presence of water and heating at a superatmospheric pressure sufficient to effect said hydrolysis. As used herein "superatmospheric" means above normal atmospheric pressure conditions, usually of the order of about 225 to about 2000 psi (1.5 x 10⁶ to about 13.7 x 10⁶ N/m²). It is important that the starting material and water be subjected to both sufficient pressures and temperatures. The above-listed ranges provide a satisfactory superfactory superatmospheric temperature and pressure. Starting material and water subjected only to heat or only to pressure will not produce hydrolysed products. The water may be distilled or deionized, but tap water is also sufficient.

The starting materials are acyclic unsaturated ketones having one or more carbon-carbon double bonds wherein at least one carbon-carbon double bond is conjugated with the carbonyl carbon-oxygen double bond. The term "acyclic" is meant to limit the class of compounds to those wherein neither the carbonyl carbon nor either of the carbons of the conjugated carbon-carbon double bond are also integral compounds of a ring structure, either cyclic or aromatic. Cyclic or aromatic substituents may exist in the same molecule, however.

Because of the nature of the hydrolysis reaction, non-reactive species present in the starting material will have little effect on the cleavage of the conjugated carbon-carbon double bond. As a result, natural sources of the acyclic unsaturated ketones may be employed to obtain products in substantial yield.

The reaction proceeds in the presence of water at a substantially neutral pH at superatmospheric pressure and temperature, for example, under pressure in the range of about 225 to about 2000 psi (1.5 x 10⁶ to about 13.7 x 10⁶ N/m²) and at a temperature in the range of about 200°C to about 325°C, preferably at least about 225 psi (1.5 x 10⁶ N/m²) and about 200°C.

Detailed operating examples are provided in parent European Patent Application No. 0346383.

It is possible to conduct the hydrolysis reaction on a batch or a continuous basis. The components can be pumped through a heated stainless steel tube having a back-pressure regulator which can maintain a pressure in the tube slightly greater than that of steam. Parameters which would affect the conversion rate are the concentration, the temperature of the tube, and the residence time in the tube.

The present invention provides a method of hydrolysing curcumin using only water, heat and pressure by either batch or continuous processes to produce desirable flavour products in good yield.

## Claims

1. A method of reacting curcumin with water to produce a flavour compound comprising at least one reaction product of curcumin and water comprising hydrolyzing curcumin by heating curcumin in the presence of water at a temperature and pressure sufficient to effect the reaction and thereby produce the compound.

2. A method of reacting curcumin as claimed in Claim 1 to produce vanillin.

3. A method as claimed in either Claim 1 or Claim 2, comprising conducting the hydrolysis reaction at substantially neutral pH in the presence of water, and heating at a superatmospheric pressure sufficient to effect the hydrolysis reaction.

4. A method as claimed in any preceding Claim, wherein the temperature is at least 200°C and the pressure is at least 225 psi (1.5 x 10⁶ N/m²).

5. A method as claimed in any preceding Claim, wherein the temperature is between 200°C and 325°C and the pressure is of the order of between 225 to 2000 psi (1.5 x 10⁶ to 14 x 10⁶ N/m²).

6. A method as claimed in any preceding Claim, wherein the volume of water exceeds the volume of the starting material.

7. A method as claimed in any preceding Claim, wherein the reaction is conducted in an autoclave or other pressure reactor.

8. A method as claimed in any one of Claims 1 to 6, wherein the hydrolysis reaction is effected continuously in a tube reactor.

## Patentansprüche

1. Verfahren zum Hervorrufen einer Reaktion von Kurkumin mit Wasser zum Herstellen einer Aromaverbindung, die mindestens ein Reaktionsprodukt des Kurkumins und Wassers enthält, umfassend: Hydrolysieren von Kurkumin durch Erhitzen von Kurkumin in Anwesenheit von Wasser bei einer Temperatur und einem Druck, die ausreichen, die Reaktion zu bewirken und dadurch die Verbindung herzustellen.

2. Verfahren zum Hervorrufen einer Reaktion von Kurkumin nach Anspruch 1 zum Herstellen von Vanillin.

3. Verfahren nach Anspruch 1 oder Anspruch 2, umfassend: Durchführen der Hydrolysereaktion bei im wesentlichen neutralem pH-Wert in Anwesenheit von Wasser und Erhitzen bei einem überatmosphärischen Druck, der ausreicht, die Hydrolysereaktion zu bewirken.

4. Verfahren nach einem der vorangehenden Ansprüche, in welchem die Temperatur mindestens 200°C und der Druck mindestens 225 psi (1,5 x 10⁶ N/m²) beträgt.

5. Verfahren nach einem der vorangehenden Ansprüche, in welchem die Temperatur zwischen 200°C und 325°C und der Druck im Bereich zwischen 225 und 2000 psi (1,5 x 10⁶ bis 14 x 10⁶ N/m²) liegt.

6. Verfahren nach einem der vorangehenden Ansprüche, in welchem das Volumen des Wassers das Volumen des Ausgangsmaterials übersteigt.

7. Verfahren nach einem der vorangehenden Ansprüche, in welchem die Reaktion in einem Autoklaven oder in einem anderen Druckreaktor ausgeführt wird.

8. Verfahren nach einem der Ansprüche 1 bis 6, in welchem die Hydrolysereaktion in einem Rohrreaktor kontinuierlich bewirkt wird.

## Revendications

1. Procédé pour faire réagir le curcumin avec de l'eau pour produire un composé parfumé comprenant au moins un composé de réaction de curcumin et d'eau, comprenant l'hydrolyse du curcumin en chauffant le curcumin en présence d'eau à une température et sous une pression suffisants pour effectuer la réaction et produire ainsi le composé.

2. Procédé pour faire réagir le curcumin selon la revendication 1, pour produire de la vanilline.

3. Procédé selon la revendication 1 ou la revendication 2, comprenant la conduite de la réaction d'hydrolyse à un pH substantiellement neutre en présence d'eau, et le chauffage sous une pression sur-atmosphérique suffisante pour effectuer la réaction d'hydrolyse.

4. Procédé selon l'une quelconque des revendications précédentes, dans lequel la température est d'au moins 200°C et la pression est d'au moins 225 psi (1,5 x 10⁶ N/m²).

5. Procédé selon l'une quelconque des revendications précédentes, dans lequel la température est entre 200°C et 325°C et la pression est de l'ordre de 225 à 2.000 psi (1,5 x 10⁶ à 14 x 10⁶ N/m²).

6. Procédé selon l'une quelconque des revendications précédentes, caractérisé en ce que le volume d'eau excède le volume de matériau de départ.

7. Procédé selon l'une quelconque des revendications précédentes, caractérisé en ce que la réaction est effectuée dans un autoclave ou autre réacteur sous pression.

8. Procédé selon l'une quelconque des revendications 1 à 6, caractérisé en ce que la réaction d'hydrolyse est effectuée en continu dans un réacteur tubulaire.
